# EUROPEAN PATENT SPECIFICATION

(11) **EP 1 451 208 B1**
(45) Date of publication and mention of the grant of the patent: **06.09.2006**
(21) Application number: 02778183.0
(22) Date of filing: 08.11.2002
(51) Int. Cl.: C07K 5/02

(54) **CONCENTRATING AQUEOUS FRACTIONS OF LISINOPRIL BY REVERSE OSMOSIS**
KONZENTRIERUNG WÄSSRIGER FRAKTIONEN VON LISINOPRIL DURCH UMGEKEHRTE OSMOSE
CONCENTRATION DE FRACTIONS AQUEUSES DE LISINOPRIL PAR OSMOSE INVERSE

(30) Priority: 26.11.2001 SI 200100300
(43) Date of publication of application: 01.09.2004
(73) Proprietor: LEK farmacevtska druzba d.d., 1526 Ljubljana (SI)
(72) Inventor: RZEN, Janez, 1000 Ljubljana (SI); SENICA, David, 1000 Ljubljana (SI); DRNOVSEK, Pavel, 1230 Domzale (SI)
(74) Representative: Dietz, Jörg-Reimar
(86) International application number: PCT/SI2002/000025
(87) International publication number: WO 2003/045981

(56) References cited:
- EP-A- 0 903 337
- US-A- 4 472 380
- US-A- 5 336 601
- US-A- 5 907 044
- WHITESIDE RG ET AL.: "Application of high speed CCC for the purification of lisinopril-diketopiperazine diastereomers" JOURNAL OF LIQUID CHROMATOGRAPHY & RELATED TECHNOLOGIES, vol. 24, no. 11-12, June 2001 (2001-06), pages 1801-1810, XP008018327
- RZEN J ET AL.: "Concentration of lisinopril purified by liquid chromatography- a comparision between reverse osmosis and evaporation" ACTA CHIMICA SLOVENICA, vol. 48, no. 4, December 2001 (2001-12), pages 597-612, XP008018264

## Description

### Technical Field of the Invention

The invention belongs to the field of chemical engineering and deals with concentrating by means of the reverse osmosis (RO).

In a narrower sense, the invention deals with a process of concentrating aqueous fractions of lisinopril by means of the reverse osmosis.

### Technical Problem

After the completed synthesis of lisinopril there are dissolved in water, in addition to the desired product, also numerous undesired impurities such as lisinopril isomers, the remains of the starting raw materials and their derivatives, by-products of reactions and similar. Most undesired impurities are removed by chromatographic purification.

In the process of purification and isolation of lisinopril it is necessary (sometimes even several times) to concentrate the aqueous fractions, wherefor a one-step vacuum circular evaporator has been used so far. The aqueous fractions were concentrated to a desired concentration of lisinopril by evaporation of water at an absolute pressure lower than 100 mbars. For the evaporation of large quantities of water, very productive evaporators are required and the operation costs are relatively high.

Our invention arises from the need for a more efficient technological solution for concentrating the aqueous fractions of lisinopril, which would be more efficient and more economical than vacuum evaporation, both prior to the final crystallization and in other steps of the synthesis process.

### Prior Art

Lisinopril is a pharmaceutically active compound from the group of angiotensin-converting enzyme (ACE) inhibitors used for the treatment of hypertension. Lisinopril dihydrate, chemically N-[N-[(1S)-carboxy-3-phenylpropyl]-L-lisyl]-L-proline dihydrate is prepared by a multi-step synthesis which is described e.g. in the article J. Org. Chem. 53 (1988) 836, in US patent 4,374,829, wherein the purification is carried out by adsorption chromatography or by lyophilization, or by some other process.
In US 4,472,380 the processes for preparing lisinopril are disclosed. Therein the reaction mixture is simply concentrated under vacuum.

In US 5,336,601 a method for an enzymatic synthesis of a peptide is disclosed. Reverse osmosis is used for removing the product from the reaction side to the product side of the membrane.

In some cases the reverse osmosis can be used for concentrating the solutions instead of classical techniques. The investment, operation and maintenance costs are lower, the reverse osmosis can operate at lower temperatures, it is simple to automate it as well as to scale it up from laboratory or pilot scales to the industrial scale. The usefulness of the reverse osmosis is limited by the chemical and physical resistance of the membranes. Depending on their materials and construction, the membranes can only be used in specific pressure, temperature and pH ranges. Too high pressures and temperatures and too low or too high pH's can irreversibly damage the membranes. The membranes can also be irreversibly damaged by some organic solvents and inorganic compounds. The reverse osmosis is not useful for concentrating solutions of solutes with a low solubility either. By a proper selection of the membranes and operating conditions, however, the reverse osmosis can be efficiently used for various purposes.

In the patent and other literature from this field, no literature source describing the use of the reverse osmosis method in a process of purification and/or isolation of lisinopril has been found.

### Description of Novel Solution with Examples

The object of the invention is a novel process for the purification and isolation of lisinopril synthesized in a multi-step synthesis. After the completed synthesis, lisinopril is chromatographically purified, the chromatographic fractions are lisinopril is chromatographically purified, the chromatographic fractions are concentrated and lisinopril is isolated by precipitation. The chromatographic fractions are concentrated to the desired concentration by the use of the reverse osmosis, which is more economical than the evaporation of large quantities of water in a simple one-stage vacuum circular evaporator. The usefulness of the reverse osmosis for concentrating was examined at various operating conditions on various scales.

After column separation, the concentrating of the aqueous solution of lisinopril by the reverse osmosis was performed on a pilot scale in a batch mode and in a continuous mode. A membrane element of the spiral-wound type with a filter surface area of 7.6 m² was used.

The operating conditions for concentrating the aqueous fractions of lisinopril were determined in the batch mode of operation. From the membrane module the concentrate flowed back into the feed tank and the permeate was removed or recycled back to column separation. The concentration range of lisinopril was from 1 g/L to 100 g/L.

The processes, the continuous and/or the batch ones, are run in a temperature range from some degrees above freezing point up to 45 °C, preferably at 5-30°C. At a prior acidification of the solution, the operating pH is in the range between 4 and 11, otherwise between 7 and 11, preferably between 9 and 10.5. The operating pressures are between between 5 and 41 bars, preferably between 10 and 55 bars. The permeate recovery (i.e. an intrinsic property of the membrane), which is the ratio between the permeate flow rate and the sum of the permeate and the concentrate flow rates (i.e. flow rate of the feed flow) expressed in %, is between 5 % and 30 %, preferably at between 10 and 25%. Depending on the operating conditions, the fluxes of the permeate were from 5 to 45 L/hm², preferably between 10-35 L/hm².

As an alternative to the membrane modules for the reverse osmosis and instead of them, there can be used nano-filtering membrane modules of various constructions such as e.g. plate and frame or spiral-wound types or the like and made of various materials (organic or inorganic).

The concentrated solution of lisinopril is crystallized according to known processes. The product is raw lisinopril, which is then converted into the final form, namely lisinopril dihydrate.

The invention is explained but in no way limited by the following Examples.

### Example 1

The chromatographic fractions, which after column separation contained 1 % of acetonitrile, 0.5 % of ammonia and 1 g/L of lisinopril, were concentrated in the batch mode in a pilot plant for the reverse osmosis. From the feed tank, the solution was pumped by high-pressure pumps into the reverse osmosis membrane module. At the beginning of concentrating, the drop of the pressure on the membrane amounted to 10 bars. During the concentrating the flow rates of the permeate and the concentrate were maintained approximately constant - the flow rate of the permeate at 140 L/h and the flow rate of the concentrate at 640 L/h. After the completed concentrating, the concentration of lisinopril in the concentrate was 100 g/L and the drop of the pressure on the membranes was 27 bars.

### Example 2

The chromatographic fractions, which after column separation contained 1 % of acetonitrile, 0.5 % of ammonia in 1 g/L of lisinopril, were concentrated in the batch mode in a pilot plant for the reverse osmosis. From the feed tank, the solution was pumped by high-pressure pumps into the reverse osmosis membrane module. At the beginning of concentrating, the drop of the pressure on the membrane amounted to 20 bars. During the concentrating the flow rates of the permeate and the concentrate were maintained approximately constant - the flow rate of the permeate at 220 L/h and the flow rate of the concentrate at 950 L/h. After the completed concentrating, the concentration of lisinopril in the concentrate was 100 g/L and the drop of the pressure on the membranes was 37 bars.

## Claims

1. A process for the purification and obtaining of lisinopril comprising
(a) synthesis of lisinopril,
(b) purification of lisinopril by means of chromatography,
(c) concentrating the aqueous solution obtained in step (b) by use of reverse osmosis, and
(d) isolation of lisinopril by crystallization,
wherein the reverse osmosis in step (c) is performed at operating temperatures up to 45 °C, pH between 4 and 11, and pressures between 5 and 41 bars.

2. The process according to claims 1 or 2, **characterized in that** the reverse osmosis is performed at operating temperatures between 5 °C and 30 °C.

3. The process according to claims 1 or 2, **characterized in that** the reverse osmosis is performed at a pH between 9 and 10.5.

4. The process according to claims 1 or 2, **characterized in that** the reverse osmosis is performed at pressures between 10 and 35 bars.

5. The process according to claims I or 2, **characterized in that** the reverse osmosis is performed at a permeate recovery between 5 % and 30 %.

6. The process according to claim 6, **characterized in that** the reverse osmosis is performed at a permeate recovery between 10 % and 25 %.

## Patentansprüche

1. Verfahren zur Reinigung und Gewinnung von Lisinopril, **gekennzeichnet durch**
(a) Synthese von Lisinopril,
(b) Reinigung von Lisinopril mittels einer Chromatographie,
(c) Konzentration der im Schritt (b) erhaltenen wässrigen Lösung **durch** Anwendung einer Umkehrosmose und
(d) Isolation von Lisinopril **durch** Kristallisation,
worin die Umkehrosmose im Schritt (c) durchgeführt wird bei Arbeitstemperaturen von bis zu 45 °C, pH Werten zwischen 4 und 11 und Drücken zwischen 5 und 41 bar.

2. Verfahren nach Anspruch 1, **dadurch gekennzeichnet, dass** die Umkehrosmose durchgeführt wird bei Arbeitstemperaturen zwischen 5 °C und 30 °C.

3. Verfahren nach Anspruch 1, **dadurch gekennzeichnet, dass** die Umkehrosmose durchgeführt wird bei pH Werten zwischen 9 und 10,5.

4. Verfahren nach Anspruch 1, **dadurch gekennzeichnet, dass** die Umkehrosmose durchgeführt wird bei Drücken zwischen 10 und 35 bar.

5. Verfahren nach Anspruch 1, **dadurch gekennzeichnet, dass** die Umkehrosmose durchgeführt wird bei einer Permeatgewinnung zwischen 5 % und 30 %.

6. Verfahren nach Anspruch 5, **dadurch gekennzeichnet, dass** die Umkehrosmose durchgeführt wird bei einer Permeatrückgewinnung zwischen 10 % und 25 %.

## Revendications

1. Procédé pour la purification et l'obtention du lisinopril, comprenant:
(a) la synthèse de lisinopril,
(b) la purification du lisinopril par chromatographie,
(c) la concentration de la solution aqueuse obtenue dans l'étape (b) par utilisation d'une osmose inverse, et
(d) l'isolement du lisinopril par cristallisation,
où l'osmose inverse de l'étape (c) est réalisée à des températures de fonctionnement allant jusqu'à 45°C, un pH compris entre 4 et 11 et des pressions comprises entre 5 et 41 bars.

2. Procédé selon les revendications 1 ou 2, **caractérisé en ce que** l'osmose inverse est réalisée à des températures de fonctionnement comprises entre 5°C et 30°C.

3. Procédé selon les revendications 1 ou 2, **caractérisé en ce que** l'osmose inverse est réalisée à un pH compris entre 9 et 10,5.

4. Procédé selon les revendications 1 ou 2, **caractérisé en ce que** l'osmose inverse est réalisée à des pressions comprises entre 10 et 35 bars.

5. Procédé selon les revendications 1 ou 2, **caractérisé en ce que** l'osmose inverse est réalisée à un taux de récupération de perméat compris entre 5% et 30%.

6. Procédé selon la revendication 6, **caractérisé en ce que** l'osmose inverse est réalisée à un taux de récupération de perméat compris entre 10% et 25%.
